# EUROPEAN PATENT SPECIFICATION

(11) **EP 2 060 273 B1**
(45) Date of publication and mention of the grant of the patent: **23.02.2011**
(21) Application number: 09153742.3
(22) Date of filing: 10.11.2004
(51) Int. Cl.: A61K 49/06, A61K 31/198, A61K 33/32

(54) **MRI contrast medium composition for oral administration**
MRI-Kontrastmediumzusammensetzung zur oralen Verabreichung
Composition de milieu de contraste pour IRM pour l'administration orale

(30) Priority: 19.12.2003 SE 0303429
(43) Date of publication of application: 20.05.2009
(62) Divisional of application: 04800307.3
(73) Proprietor: CMC Contrast AB, 223 70 Lund (SE)
(72) Inventor: Johansson, Nils-Olof, SE-181 66, LIDINGÖ (SE)
(74) Representative: Presland, Torbjörn

(56) References cited:
- WO-A-96/05867
- WO-A-97/02842
- WO-A-98/11922
- THOMSEN H S ET AL: "Increased manganese concentration in the liver after oral intake" ACADEMIC RADIOLOGY, RESTON, VA, US, vol. 11, no. 1, 1 January 2004 (2004-01-01), pages 38-44, XP002984165 ISSN: 1076-6332

## Description

### FIELD OF THE INVENTION

The present invention relates to magnetic resonance imaging (MRI), more specifically to the oral administration of manganese contrast medium compositions for imaging of.the liver and a method for MRI of a liver using the manganese contrast medium composition.

### BACKGROUND OF THE INVENTION

Magnetic resonance imaging (MRI) is now well established as a medical diagnostic tool. The ability of the technique to generate high quality images and to differentiate between soft tissues without requiring the patient to be exposed to ionizing radiation has contributed to this success.

Manganese is a well-known paramagnetic contrast medium useful for MRI of soft tissues in the body. When administered intravenously as a contrast medium, manganese may however be teratogenic at clinical dosages. Administered intravenously, manganese is also known to interfere with the normal functioning of the heart by replacement of calcium in the muscular cell of the heart. To avoid this problem Mn bound in chelate complexes has been used; however the chelation prevents or reduces the enhancement of Mn binding with tissues.

EP 0 308 983 A2 thus proposes a contrast medium for parenteral application for imaging of the heart and liver in the form of a non-chelate coordination compound to reduce toxicity. Here Mn is bound in a complex, i.e. a coordination complex, to avoid the toxic effect of free Mn. The proposed compound has the formula Mn(II) (H₂O)ₘAlₙA2ₒA3ₚA4_{q})+^{a}(Y,Zᵣ)^{-a}, wherein A1, A2, A3 and A4 are the same or different amino-substituted carboxylic acid groups having from 2 to 18 carbons; Y and Z are each the same or a different anion of a pharmaceutically acceptable inorganic acid or an organic carboxylic acid having from 2 to 18 carbons; a is the valence of the ions; m, n, o, p and q are each 1 or 0, (m + n + o + p + q) = 4; and..r is 1, and if Y is a multivalent anion, r is 0 or 1.

EP 0 308 983 A2 discloses organ specifically designed compositions for administration by injection and selected to carry the coordination compound to the portion of the body to be imaged by the MR device.

In order to further reduce the risk of dangerous or even fatal effect on the heart, oral administration of manganese has previously been proposed. A result of the vascularisation of the upper gastrointestinal tract is that orally administered material taken up into the blood from the gut is adsorbed in the liver before passing to the heart. In the case of manganese, absorption by the hepatocytes in the liver prevents cardiotoxic levels of manganese reaching the heart. This hepatocyte uptake of manganese has led to the proposed use of orally administered manganese as a liver imaging contrast medium.

WO 96/05867 discloses a contrast medium composition comprising a physiologically tolerable manganese compound, an uptake promoter and a physiologically tolerable carrier or excipient, having a manganese concentration of at least 0.3 mM or being in a dosage unit form containing at least 300 µmol manganese, wherein the uptake promoter comprises a physiologically tolerable reducing compound containing an α-hydroxy ketone group, a physiologically tolerable acid containing α- and/or β-hydroxy or amino groups, or a salt thereof, and/or vitamin D. The disclosure clearly indicates that improved uptake of a manganese will be obtained by using an uptake promoter in molar excess over manganese.

WO 97/02842 discloses the use of a Mn-contrast medium containing a promoter for oral administration for imaging of the stomach, liver, bile duct and gall bladder. The promoter is in the form of at least one amino acid and/or a vitamin D. However, nor does this document teach any advantage of avoiding substantial formation of coordination compounds between manganese and uptake promoter.

WO 98111922 discloses the use of a physiologically tolerable manganese compound or a salt thereof, in combination with a second contrast agent, preferably one which is retained within the gut and there exhibits a negative contrast effect, in the manufacture of an enterally, e.g. orally or rectally, administrable MRI contrast medium composition for use in a method of functional imaging of the gastrointestinal tract of a human or non-human animal body.

### SUMMARY OF THE INVENTION

It has now surprisingly been found that by increasing previously taught molar ratios of Mn to amino acid it is possible to improve the liver uptake of manganese, and such improvement is independent of whether vitamin D₃ is present or not.

In this connection it is important to note that even a relatively small increase in the amount of manganese present in the liver tissue will result in a significant enhancement of the image.

In one aspect the present invention provides for the use of a physiologically acceptable manganese (II) compound and an uptake promoter in the form of one or more amino acids for the manufacture of an MRI contrast composition for oral administration and MRI examination of the liver, in a ratio of Mn/promoter higher than that at which coordination compounds between Mn and promoter are formed to a substantial degree, wherein the molar ratio of Mn to promoter is in the range of from 2:1 to 3:1.

In a second aspect the invention provides for an MRI contrast medium composition for oral administration for examination of the liver, comprising as an active ingredient a physiologically acceptable manganese (II) compound and an uptake promoter comprising one or more amino acids, wherein Mn and the promoter are used in a molar ratio higher than that at which coordination compounds between Mn and promoter are formed to a substantial degree, wherein the molar ratio of Mn to promoter is in the range of from 2:1 to 3:1. In a third aspect the invention provides for an MRI contrast medium kit comprising a first container acommodating a physiologically acceptable manganese (II) compound, a second container accomodating an uptake promoter comprising one or more amino acids, and, optionally, instructions for use of the kit, the molar ratio of Mn/promoter being within the range of 2:1 to 3:1.

In a fourth aspect the invention provides for a method for MRI of a mammalian liver using a contrast medium composition as defined above, said method comprising oral administration of an effective amount of a contrast composition as described above to a mammal, including man, in need of such an MRI.

### DETAILED DESCRIPTION OF THE INVENTION

The prior art describes on the one hand the necessity of providing manganese in the form of coordination complexes to avoid toxic effects by injection and, on the other hand, the advantage of using the uptake promoter in a molar excess over the manganese when using oral administration.

The main object of the present invention is to provide contrast medium compositions for oral administration of manganese for improved imaging of the liver due to increased absorption from the gastro-intestinal tract.

The present invention is taking advantage of the novel finding that ratios of manganese/uptake promoter which form coordination complexes are not very effective in regard to adsorption in the liver. Accordingly, another object of the present invention is to provide compositions, wherein manganese and uptake promoter are present in such portions as not to form coordination complexes to any significant extent. Expressed in another way the ratio between manganese and promoter shall be higher than that at which coordination compounds between manganese and promoter are formed to a substantial degree. In this context "substantial" is to be interpreted preferably as major, i.e. leaving more than 50% of the manganese ions non-coordinated and available for absorption.

Thus, the present invention is based on the surprising finding that coordination compounds involving manganese and uptake promoter are to be avoided in order that improved adsorption of manganese in the liver will be obtained.

In the composition according to the invention, the molar ratio of manganese to uptake promoter is from about 2:1 to about 3:1.

The preferred dosage of the composition according to the present invention will vary according to a number of factors, such as the age, weight and species of the subject, and the particular uptake promoter used. Conveniently, the dosage of manganese will normally be in the range of from about 25 to about 150 µmol/ kg body weight. Preferably the dosage of manganese will be in a range of from about 50 to about 125 µmol/ kg body weight, and more preferably the dosage of manganese will be in the range of from about 50 to about 100 µmol/ kg body weight.

The contrast medium composition according to the invention may comprise a manganese compound together with a mixture of two or more uptake promoters, e.g. a mixture of several amino acids.

Compounds which have been found to be suitable for use as uptake promoters include all physiologically acceptable amino acids. Amino acids which are effective as uptake promoters in the compositions of the invention include all the native amino acids, i.e. alanine, valine, leucine, tryptophan, methionine, isoleucine, proline, phenylalanine, serine, glycine, threonine, cysteine, asparagine, glutamine, tyrosine, aspartic.acid, glutamic acid, arginine, lycine and histidine.

A preferred group of amino acids for use as uptake promoters in the compositions of this invention is selected from neutral amino acids including asparagine, and aspartic acid. Particularly preferred amino acids are asparagine, aspartic acid and alanine, especially L-alanine.

The invention is preferably practised while using the amino acid(s) as the only promoter(s), but this does not exclude the use of said amino acid(s) together with any other common promoter(s), e.g. vitamin D₃.

Alanine has been used as an example in the present invention. WO 96/05867 shows a general effect of other amino acids, such as glycine, valine, glutamine, aspartic acid, glutamic acid, lysine, arginine, cysteine and methionine. Decisive for the choice of amino acid or amino acids are the price and the stability as well as the taste, appearance and odour of the amino acid to have a product acceptable to the patients.

Paramagnetic materials, such as manganese ions, may act as either positive or negative MRI contrast agents depending upon a number of factors, including the concentration of the ions at the imaging site and the magnetic field strength used in the imaging procedure. At the concentrations of manganese contemplated for use in the compositions of the invention, the manganese-containing contrast medium will in general function as a positive contrast medium.

The compositions of the invention are particularly suited to use as a dispersion in an aqueous medium. For such a purpose the composition may be administered into the gastrointestinal tract orally or via a gastric tube.

It is possible to formulate the contrast medium immediately or shortly prior to administration by mixing the uptake promoter with the manganese compound.

As the contrast medium is to be administered orally a patient can administer it himself. The patient is therefore not obliged to stay in the hospital for several hours before being scanned. He can take the oral medium himself without need for medical assistance.

The contrast medium composition of the invention may include components other than the uptake promoter, and the manganese compound, for example conventional pharmaceutical formulation aids, such as wetting agents, buffers, disintegrants, binders, fillers, flavouring agents and liquid carrier media, such as sterile water, water/ethanol.

The pH of the composition is preferably in the acidic range, e.g. 2 to 7, and while the uptake promoter may itself serve to yield a composition with this pH, buffers or pH adjusting agents may be used.

The contrast media may be formulated in any edible/drinkable medium and in conventional pharmaceutically administrable forms, such as tablets, capsules, powders, solutions, dispersions, or syrups. Since the contrast medium is to be administered orally, a patient can administer the contrast medium himself prior to scanning.

The manganese compound, which for oral administration is preferably dissolved or suspended in water, may for example be in the form of a salt, or may be a mixture of different salts. Particularly preferred are salts in which the manganese is present as Mn(II) rather than Mn(III) since the former has a better adsorption profile and is thus more efficient as an MR contrast medium for the liver.

Examples of manganese compounds preferred for use in accordance with the invention include pharmaceutically acceptable salts, e.g. manganese chloride, manganese ascorbate, manganese kojate, manganese salicylate and manganese gluconate, especially the chloride.

The invention will now be further described by examples which illustrate the effectiveness of the uptake of manganese for various concentrations of uptake promoter.

The compositions that do not fall under the scope of the claims do not form part of the present invention and are of comparative nature.

### Example 1

The study was conducted at Scantox, Hestehavevej 6A, DK-4623 Lille Skensved, Denmark.

The objective of the studies was to evaluate the uptake of manganese chloride in rats.by measurement of the manganese content in the liver as a comparison example.

The experiment was performed in 55 outbred Sprague Dawley rats from Taconic M&B A/S, Denmark. The animals in the study were allocated to 11 groups. The test composition was administered once orally by gavage. Animals in Group 1 (control) were only treated with sterile water. Group 2 received only 100 µmol MnCl₂/kg. The other nine treated groups received concurrent oral doses of 100 µmol MnCl₂/kg (Group 3 to 11) and/or 450, 300 and 150 µmol alanine/kg and/or 20, 40 and 60 IU vitamin D₃/kg in different permutations (table 1).

Three hours after dose administration, the animals were killed and the livers were weighed and retained frozen. The samples were subsequently homogenised and analysed for manganese concentrations.

The highest group mean amount of manganese was seen in group 5, receiving respectively 150 µmol/kg alanine and 60 IU vitamin D₃/kg, indicating that a low amount of alanine, i.e. 150 µmol/kg is increasing the manganese uptake in the liver, compared to groups 3 and 4, receiving 450 and 300 µmol/kg alanine, respectively and the same amount of vitamin D₃ (see table 1). The results in table 1 show clearly that there is a significant increase in manganese uptake from the gut with.decreasing amounts of alanine. For the three subgroups 60, 40 and 20 IU vitamin D₃/kg bodyweight, the.best manganese uptake is seen with the lowest alanine amount, i.e. 150 µmol/kg bodyweight (group 5 compared to 3 and 4, group 8 compared to group 6 and 7, group 11 compared to group 9 and 10). However a vitamin D₃ content of 40 IU/kg bodyweight shows no difference between group 7 and 8, which suggest that in this level of vitamin D₃ there is now significant difference between 300 and 150 µmol/kg alanine

**Table 1 (grouped according to vit. D₃ content)**

| Group | Treatment | | | Mol. Ratio Mn:Ala | Liver Mean Mn nmol/g |
|---|---|---|---|---|---|
| | MnCl₂ (µmol/kg b.w.) | Alanine (µmol/kg b.w.) | Vitamin D₃ (IU/kg b.w.) | | |
| 1 | 0 | 0 | 0 | - | 41.4 |
| 2 | 100 | 0 | 0 | - | 44.4 |
| 3 | 100 | 450 | 60 | 1:4.5 | 61.1 |
| 4 | 100 | 300 | 60 | 1:3 | 61.8 |
| 5 | 100 | 150 | 60 | 1:1.5 | 76.1 |
| 6 | 100 | 450 | 40 | 1:4.5 | 66.4 |
| 7 | 100 | 300 | 40 | 1:3 | 73.8 |
| 8 | 100 | 150 | 40 | 1:1.5 | 73.7 |
| 9 | 100 | 450 | 20 | 1:4.5 | 68.4 |
| 10 | 100 | 300 | 20 | 1:3 | 69.8 |
| 11 | 100 | 150 | 20 | 1:1.5 | 72.6 |

Taken together, table 1 shows that when the ratio of Mn:ala is 1:4.5, the Mn liver uptake is enhanced by addition of vit D₃ (group 3, 6 and 9). However when the ratio of Mn:ala is 1:1.5 addition of vit.D₃ in a high amount (i.e. 60 or 40 IU/kg b.w.) is not needed in order to obtain fairly good images (group 5, 8 and 11).

### Example 2

The study was conducted as described in example 1. However the experiment was performed in 55 outbred Sprague Dawley rats from Taconic M&B A/S, Denmark. The animals in the study were allocated to 11 groups. The test composition was administered orally by gavage. Animals in Group 1 (control) were only treated with sterile water. Group 2 received only 100 µmol MnCl₂/kg. The other nine treated groups received concurrent oral doses of 100 µmol MnCl₂/kg (Group 3 to 11), 150, 75 and 37.5 µmol alanine/kg and 20, 10 and 5 IU vitamin D₃/kg in different permutations (table 2A).

Three hours after dose administration, the animals were killed and the livers were weighed and retained frozen. The samples were subsequently homogenised and analysed for manganese concentrations.

Manganese concentrations in the liver were higher in groups 3 to 11 compared to Groups 1 and 2. This correlates well with the fact that Groups 1 and 2 received either 0 or 100 µmol MnCl₂/kg without addition of uptake promoters. The highest group mean amount of manganese was seen in group 6 to 11, receiving respectively 75 and 37.5 µmol/kg alanine, indicating that a high amount of alanine, i.e. 150 µmol/kg is not increasing the manganese uptake in the liver (group 3 to 5) compared to the alanine amounts provided to group 6 to 11 (see table 2A (or 2B, NB: different grouping)). The results clearly show that there is a significant increase in manganese uptake from the gut with the use of promoters wherein the molecular ratio between Mn and amino acid, for example alanine is higher than 1:1.

**Table 2A (Grouped according to alanine content)**

| Group | Treatment | | | Mol. Ratio Mn:Ala | Liver Mean Mn nmol/g |
|---|---|---|---|---|---|
| | MnCl₂ (µmol/kg b.w.) | Alanine (µmol/kg b.w.) | Vitamin D₃ (IU/kg b.w.) | | |
| 1 | 0 | 0 | 0 | - | 34.6 |
| 2 | 100 | 0 | 0 | - | 63.4 |
| 3 | 100 | 150 | 20 | 1:1.5 | 69.9 |
| 4 | 100 | 150 | 10 | 1:1.5 | 72.7 |
| 5 | 100 | 150 | 5 | 1:1.5 | 68.4 |
| 6 | 100 | 75 | 20 | 1:0.75 | 69.2 |
| 7 | 100 | 75 | 10 | 1:0.75 | 73.8 |
| 8 | 100 | 75 | 5 | 1:0.75 | 72.0 |
| 9 | 100 | 37.5 | 20 | 1:0.375 | 72.6 |
| 10 | 100 | 37.5 | 10 | 1:0.375 | 79.8 |
| 11 | 100 | 37.5 | 5 | 1:0.375 | 73.1 |

**Table 2B (grouped according to Vit. D₃ content)**

| Group | Treatment | | | Mol. Ratio Mn:Ala | Liver Mean Mn nmol/g |
|---|---|---|---|---|---|
| | MnCl₂ (µmol/kg b.w.) | Alanine (µmol/kg b.w.) | Vitamin D₃ (IU/kg b.w.) | | |
| 1 | 0 | 0 | 0 | - | 34.6 |
| 2 | 100 | 0 | 0 | - | 63.4 |
| 3 | 100 | 150 | 20 | 1:1.5 | 69.9 |
| 6 | 100 | 75 | 20 | 1:0.75 | 69.2 |
| 9 | 100 | 37.5 | 20 | 1:0.375 | 72.6 |
| 4 | 100 | 150 | 10 | 1:1.5 | 72.7 |
| 7 | 100 | 75 | 10 | 1:0.75 | 73.8 |
| 10 | 100 | 37.5 | 10 | 1:0.375 | 79.8 |
| 5 | 100 | 150 | 5 | 1:1.5 | 68.4 |
| 8 | 100 | 75 | 5 | 1:0.75 | 72.0 |
| 11 | 100 | 37.5 | 5 | 1:0.375 | 73.1 |

### Example 3

The study was conducted as described in Ex.1 and 2. However the experiment was performed in 40 male outbred Sprague Dawley rats from Taconic M&B A/S, Denmark. The animals in the study were allocated to 8 groups. The test article was administered once orally by gavage. Animals in Group 1 (control) were only treated with sterile water. Group 2 received only 100 µmol MnCl₂/kg b.w. The other six treated groups received concurrent oral doses of 50 µmol (Group 3) or 100 µmol MnCl₂/kg (Group 4 to 8), 25, 50, 200 and 200 µmol Alanine/kg and 0 or 10 IU vitamin D₃/kg in different permutations (see also table 3).

Manganese concentrations in the liver were higher in groups 2, 4, 5, 6, 7 and 8 compared to Groups 1 and 3. This correlates well with the fact that Groups 1 and 3 received the lowest amount of manganese i.e. 0 or 50 µmol MnCl₂/kg, respectively. The highest group mean amount of manganese was seen in group 5 and 7 (Table 3).

It is worth noting that group 3 and 4 showed a remarkable difference in manganese uptake, 95.3 and 110.3 respectively, even though that the molecular ratio between alanine and manganese was the same in the 2 groups, i.e. 2:1. This shows that the amount of manganese in the solution is important for an improved manganese uptake (Table 3). 100 µmol/kg bodyweight is far better than 50 µmol/kg bodyweight.

Furthermore, the manganese uptake indicates that alanine also has to be provided in a certain amount, i.e. > 25 µmol/kg b.w. since the manganese uptake in group 5 giving 50 µmol/kg alanine is better than in group 6 only provided 25 µmol/kg, i.e 128.8 nmol/g and 115.9 nmol/g respectively (Table 3).

In conclusion, Group 5 and 7 (treated with a combination of 100 µmol MnCl₂/kg and 50 µmol alanine/kg and, respectively 10 IU vitamin D₃) was the groups having the highest amount of manganese uptake in the liver. However, also compositions without vitamin D₃ show good results.

**Table 3**

| Group | Treatment | | | Mol. Ratio Mn:Ala | Liver Mean Mn nmol/g |
|---|---|---|---|---|---|
| | MnCl₂ (µmol/kg b.w.) | Alanine (µmol/kg b.w.) | Vitamin D₃ (IU/kg b.w.) | | |
| 1 | 0 | 0 | 0 | - | 45.0 |
| 2 | 100 | 0 | 0 | - | 114.0 |
| 3 | 50 | 100 | 0 | 1:2 | 95.3 |
| 4 | 100 | 200 | 0 | 1:2 | 110.3 |
| 5 | 100 | 50 | 0 | 1:0.5 | 128.8 |
| 6 | 100 | 25 | 0 | 1:0.25 | 115.9 |
| *7 | 100 | 50 | 10 | 1:0.5 | 133.8 |
| 8 | 100 | 25 | 10 | 1:0.25 | 117.8 |

| | | | | | |
|---|---|---|---|---|---|
| group 7: 1 animal excluded since no Mn was taken up | | | | | |

### Discussion:

The above specific examples clearly indicate the advantage of avoiding substantial formation of coordination compounds between manganese and uptake promoter in order to obtain improved gastro-intestinal uptake of manganese for administration to the liver following oral administration of the contrast medium composition. This finding is contrary to what could be expected in a consideration of the state of the art.

The main aspects of the present invention are disclosed in the following itemized list.
Item 1. The use of a physiologically acceptable manganese (II) compound and an uptake promoter in the form of one or more amino acids for the manufacture of an MRI contrast composition for oral administration and MRI examination of the liver, in a ratio of Mn to promoter higher than that at which coordination compounds between Mn and promoter are formed to a substantial degree, wherein the molar ratio of Mn to promoter is in the range of from 2:1 to 3:1.
Item 2. The use according to any one of the preceding items, wherein the dosage of manganese is in the range of from about 25 to about 150 µmol/ kg body weight.
Item 3. The use according to item 2, wherein the dosage of manganese is in the range of from about 50 to about 125 µmol/ kg body weight.
Item 4. The use according to item 3, wherein the dosage of manganese is in the range of from about 50 to about 100 µmol/ kg body weight
Item 5. The use according to any one of the preceding items, wherein the uptake promoter is selected from the group consisting of alanine, valine, leucine, tryptophan, methionine, isoleucine, proline, phenylalanine, serine, glycine, threonine, cysteine, asparagine, glutamine, tyrosine, aspartic acid, glutamic acid, arginine, lycine and histidine.
Item 6. The use according to item 5, wherein said promoter is selected from neutral amino acids including asparagine and aspartic acid.
Item 7. The use according to item 6, wherein said promoter is L-alanine
Item 8. An MRI contrast medium composition for oral administration for examination of the liver comprising as an active ingredient a physiologically acceptable manganese (II) compound and an uptake promoter comprising one or more amino acids wherein Mn and the promoter are used in a molar ratio higher than that at which coordination compounds between Mn and promoter are formed to a substantial degree, wherein the molar ration of Mn to promoter is in the range of from 2:1 to 3:1.
Item 9. A composition according to item 8, wherein the dosage of manganese is as defined in any one of items 2-4.
Item 10. A composition according to any one of items 8 or 9, wherein said uptake promoter is as defined in any one of items 5-7.
Item 11. An MRI contrast medium kit comprising a first container accomodating a physiologically acceptable manganese (II) compound, and a second container accomodating an uptake promoter comprising one or more amino acids, and optionally, instructions for the use of the kit, the molar ratio of Mn to promoter being within the range of 2:1 to about 3:1.
Item 12. A kit according to item 17, wherein the dosage of manganese and/or said uptake promoter is (are) as defined in any one of items 2-7.
Item 13. A method for MRI of a mammalian liver using an MRI contrast medium composition according to any one of items 8-10, said method comprising oral administration of an effective amount of said contrast medium composition.

## Claims

1. The use of a physiologically acceptable manganese (II) compound and an uptake promoter in the form of one or more amino acids for the manufacture of an MRI contrast composition for oral administration and MRI examination of the liver, in a ratio of Mn to promoter higher than that at which coordination compounds between Mn and promoter are formed to a substantial degree, wherein the molar ratio of Mn to promoter is in the range of from 2:1 to 3:1.

2. The use according to claim 1, wherein the dosage of manganese is in the range of from about 25 to about 150 µmol/kg body weight, preferably in the range of from about 50 to about 125 µmol/kg body weight, most preferably in the range of from about 50 to about 100 µmol/kg body weight.

3. The use according to any one of the preceding claims, wherein the uptake promoter is selected from the group consisting of alanine, valine, leucine, tryptophan, methionine, isoleucine, proline, phenylalanine, serine, glycine, threonine, cysteine, asparagine, glutamine, tyrosine, aspartic acid, glutamic acid, arginine, lycine and histidine.

4. The use according to claim 3, wherein said promoter is selected from neutral amino acids including asparagine and aspartic acid.

5. The use according to claim 4, wherein said promoter is L-alanine.

6. An MRI contrast medium composition for oral administration for examination of the liver comprising as an active ingredient a physiologically acceptable manganese (II) compound and an uptake promoter comprising one or more amino acids wherein Mn and the promoter are used in a molar ratio higher than that at which coordination compounds between Mn and promoter are formed to a substantial degree, wherein the molar ratio of Mn to promoter is in the range of from 2:1 to 3:1.

7. A composition according to claim 6, wherein the dosage of manganese is in the range of from about 25 to about 150 µmol/kg body weight, preferably in the range of from about 50 to about 125 µmol/kg body weight, most preferably in the range of from about 50 to about 100 µmol/kg body weight.

8. A composition according to claim 6 or 7, wherein said uptake promoter is as defined in any one of claims 3 to 5.

9. An MRI contrast medium kit comprising a first container accommodating a physiologically acceptable manganese (II) compound, and a second container accommodating an uptake promoter comprising one or more amino acids, and optionally, instructions for the use of the kit, the molar ratio of Mn to promoter being within the range of 2:1 to 3:1.

10. A kit according to claim 9, wherein the dosage of manganese is in the range of from about 25 to about 150 µmol/kg body weight, preferably in the range of from about 50 to about 125 µmol/kg body weight, most preferably in the range of from about 50 to about 100 µmol/kg body weight.

11. A kit according to claim 9 or 10, wherein said uptake promoter is as defined in any one of claims 3 to 5.

12. A method for MRI of a mammalian liver using an MRI contrast medium composition according to any one of claims 6 to 8, said method comprising oral administration of an effective amount of said contrast medium composition.

## Patentansprüche

1. Verwendung einer physiologisch akzeptablen Mangan-(II)-Verbindung und eines Aufnahmepromotors in Form einer oder mehrerer Aminosäuren zur Herstellung einer MRT-Kontrastzusammensetzung zur oralen Verabreichung und MRT-Untersuchung der Leber, in einem Verhältnis von Mn zum Promotor, das höher ist als das, mit dem Koordinationsverbindungen zwischen Mn und Promotor maßgeblich gebildet werden, wobei das Molverhältnis von Mn zu Promotor im Bereich von 2:1 bis 3:1 liegt.

2. Verwendung gemäß Anspruch 1, wobei die Dosierung von Mangan im Bereich von etwa 25 bis etwa 150 µmol/kg Körpergewicht, vorzugsweise im Bereich von etwa 50 bis etwa 125 µmol/kg Körpergewicht, am bevorzugtesten im Bereich von etwa 50 bis etwa 100 µmol/kg Körpergewicht liegt.

3. Verwendung gemäß einem der vorhergehenden Ansprüche, wobei der Aufnahmepromotor ausgewählt ist aus der Gruppe bestehend aus Alanin, Valin, Leucin, Tryptophan, Methionin, Isoleucin, Prolin, Phenylalanin, Serin, Glycin, Threonin, Cystein, Asparagin, Glutamin, Tyrosin, Asparaginsäure, Glutaminsäure, Arginin, Lysin und Histidin.

4. Verwendung gemäß Anspruch 3, wobei der Promotor ausgewählt ist aus neutralen Aminosäuren, einschließlich Asparagin und Asparaginsäure.

5. Verwendung gemäß Anspruch 4, wobei der Promotor L-Alanin ist.

6. MRT-Kontrastmedium-Zusammensetzung zur oralen Verabreichung zur Untersuchung der Leber, welche als aktiven Inhaltsstoff eine physiologisch akzeptable Mangan-(II)-Verbindung und einen Aufnahmepromotor, der eine oder mehrere Aminosäuren umfasst, enthält, wobei das Mn und der Promotor in einem Molverhältnis verwendet werden, das höher ist als das, mit dem Koordinationsverbindungen zwischen Mn und Promotor maßgeblich gebildet werden, wobei das Molverhältnis von Mn zu Promotor im Bereich von 2:1 bis 3:1 liegt.

7. Zusammensetzung gemäß Anspruch 6, wobei die Dosierung von Mangan im Bereich von etwa 25 bis etwa 150 µmol/kg Körpergewicht, vorzugsweise im Bereich von etwa 50 bis etwa 125 µmol/kg Körpergewicht, am bevorzugtesten im Bereich von etwa 50 bis etwa 100 µmol/kg Körpergewicht liegt.

8. Zusammensetzung gemäß Anspruch 6 oder 7, wobei der Aufnahmepromotor wie in einem der Ansprüche 3 bis 5 definiert ist.

9. MRT-Kontrastmedium-Kit, umfassend einen ersten Behälter, in dem eine physiologisch akzeptable Mangan-(II)-Verbindung untergebracht ist, und einen zweiten Behälter, in dem ein Aufnahmepromotor untergebracht ist, welcher eine oder mehrere Aminosäuren umfasst, und wahlweise eine Anleitung zur Verwendung des Kits, wobei das Molverhältnis von Mn zu Promotor innerhalb des Bereichs von 2:1 bis 3:1 liegt.

10. Kit gemäß Anspruch 9, wobei die Dosierung von Mangan im Bereich von etwa 25 bis etwa 150 µmol/kg Körpergewicht, vorzugsweise im Bereich von etwa 50 bis etwa 125 µmol/kg Körpergewicht, am bevorzugtesten im Bereich von etwa 50 bis etwa 100 µmol/kg Körpergewicht liegt.

11. Kit gemäß Anspruch 9 oder 10, wobei der Aufnahmepromotor wie in einem der Ansprüche 3 bis 5 definiert ist.

12. Verfahren zur MRT einer Säugetierleber unter Verwendung einer MRT-Kontrastmedium-Zusammensetzung gemäß einem der Ansprüche 6 bis 8, wobei das Verfahren die orale Verabreichung einer wirksamen Menge der Kontrastmedium-Zusammensetzung umfasst.

## Revendications

1. Utilisation d'un composé de manganèse (II) physiologiquement acceptable et d'un promoteur d'absorption sous la forme d'un ou de plusieurs acides aminés pour la fabrication d'une composition de contraste pour IRM pour une administration par voie orale et pour un examen par IRM du foie, en un rapport du Mn sur le promoteur supérieur à celui auquel des composés de coordination entre le Mn et le promoteur se forment à un degré substantiel, le rapport molaire du Mn sur le promoteur se trouvant dans la plage de 2/1 à 3/1.

2. Utilisation selon la revendication 1, la dose de manganèse se trouvant dans la plage d'environ 25 à environ 150 µmol/kg de poids corporel, de préférence dans la plage d'environ 50 à environ 125 µmol/kg de poids corporel, idéalement dans la plage d'environ 50 à environ 100 µmol/kg de poids corporel.

3. Utilisation selon l'une quelconque des revendications précédentes, le promoteur d'absorption étant choisi dans le groupe constitué par l'alanine, la valine, la leucine, le tryptophane, la méthionine, l'isoleucine, la proline, la phénylalanine, la sérine, la glycine, la thréonine, la cystéine, l'asparagine, la glutamine, la tyrosine, l'acide aspartique, l'acide glutamique, l'arginine, la lysine et l'histidine.

4. Utilisation selon la revendication 3, ledit promoteur étant choisi parmi les acides aminés neutres comprenant l'asparagine et l'acide aspartique.

5. Utilisation selon la revendication 4, ledit promoteur étant la L-alanine.

6. Composition de milieu de contraste pour IRM destinée à une administration par voie orale pour l'examen du foie qui comprend en tant que principe actif un composé de manganèse (II) physiologiquement acceptable et un promoteur d'absorption qui comprend un ou plusieurs acides aminés, le Mn et le promoteur étant utilisés en un rapport molaire supérieur à celui auquel des composés de coordination entre le Mn et le promoteur se forment à un degré substantiel, le rapport molaire du Mn sur le promoteur se trouvant dans la plage de 2/1 à 3/1.

7. Composition selon la revendication 6, la dose de manganèse se trouvant dans la plage d'environ 25 à environ 150 µmol/kg de poids corporel, de préférence dans la plage d'environ 50 à environ 125 µmol/kg de poids corporel, idéalement dans la plage d'environ 50 à environ 100 µmol/kg de poids corporel.

8. Composition selon la revendication 6 ou 7, ledit promoteur d'absorption étant tel que défini dans l'une quelconque des revendications 3 à 5.

9. Kit de milieu de contraste pour IRM qui comprend un premier récipient contenant un composé de manganèse (II) physiologiquement acceptable, et un second récipient contenant un promoteur d'absorption comprenant un ou plusieurs acides aminés et facultativement un mode d'emploi pour le kit, le rapport molaire du Mn sur le promoteur se trouvant dans la plage de 2/1 à 3/1.

10. Kit selon la revendication 9, la dose de manganèse se trouvant dans la plage d'environ 25 à environ 150 µmol/kg de poids corporel, de préférence dans la plage d'environ 50 à environ 125 µmol/kg de poids corporel, idéalement dans la plage d'environ 50 à environ 100 µmol/kg de poids corporel.

11. Kit selon la revendication 9 ou 10, ledit promoteur d'absorption étant tel que défini dans l'une quelconque des revendications 3 à 5.

12. Procédé d'IRM d'un foie de mammifère en utilisant une composition de milieu de contraste pour IRM selon l'une quelconque des revendications 6 à 8; ledit procédé comprenant l'administration par voie orale d'une quantité efficace de ladite composition de milieu de contraste.
